# EUROPEAN PATENT APPLICATION

(11) **EP 3 875 064 A1**
(43) Date of publication of application: **08.09.2021**
(21) Application number: 21159322.3
(22) Date of filing: 25.02.2021
(51) Int. Cl.: A61F 5/02

(54) **BACK BRACE**

(30) Priority: 02.03.2020 US 202016806006
(71) Applicant: Buescher, Tracy, New York, NY 10075 (US)
(72) Inventor: Buescher, Tracy, New York, NY 10075 (US)
(74) Representative: Findlay, Alice Rosemary

(57) **Abstract**

A thin back brace comprising a flexible, elastic, elongated panel, sheathed in knit neoprene or spacer fabric including antimicrobial silver containing fiber, extending in a longitudinal direction and adapted to be wrapped about the lumbar region and abdomen comprising a central zone and outlying zones on opposite edges of the central zone, the outlying zones each having elastic strip portions lying along directions that are skewed relative to the longitudinal direction such that when the brace is worn the strips are oriented upwardly with increasing proximity to the central zone such that they resist ride-up of the central zone by developing an increase in tension and a downward force component on the central zone.

## Description

Back pain is a common ailment among humans. In some cases relief can be obtained by use of a well-fitted and competent brace applied to the lumbar region of the human spine.

After headaches, the most common reason people go to doctors is for lower back pain. Prior art back braces, while often expensive, can be cumbersome or bulky so that a person concerned with their appearance may be reluctant or refuse to wear such a brace in public. Medical doctors observe that people don't wear back braces because of the way they look, not because they don't work. A common question when going to a pain management doctor for the back is: Don't you have a better looking brace? Intermittent use of a brace while an abnormal condition exists, may not be as effective as continuous use for a lasting correction. Another frequent criticism of prior art back braces is that they are prone to ride-up out of position from the hips when a person sits or engages in other common body movements.

The invention at least in the preferred embodiments provides a highly conformable, low bulk back brace with a construction that is comfortable to wear and durable in use and that can be produced at moderate cost. The back brace serves the purpose of giving back relief but also looks nice at the same time, thereby making it more apt to be worn as prescribed. The brace is a composite of elastic bands, semi-flexible stays or boning and highly stretchable, soft covering fabric. Preferably, the covering fabric includes a knit component with a co-extruded polyester or nylon fiber containing antimicrobial silver particles, such as that marketed under the U.S. Registered Trademark XT2® by Noble Fiber Technologies. The brace resists shifting or riding up from the lumbar spine section of the wearer.

A majority of lower back pain emanates from levels L3-4, L4-5 and L5-5. The disclosed brace is for lower back pain and relief in these specific areas of pain origination.

The disclosed embodiment of the back brace preferably comprises a core of elastic bands and extensible fish line fabric carrying semi-rigid stays and inner and outer layers of knitted fabric covering the core. The brace components are sewn together in a flat, belt-like configuration with a free length corresponding to the girth of a wearer. To be worn, the center of the brace is simply aligned with the spine in the lumbar region, wrapped around the waist, drawn snug and held with moderate elastic tension by coupling the ends of the brace together. The brace compressive force on the lumbar region can be "fine-tuned" by snugging up and an auxiliary elastic belt attached to the outer cover layer.

The ability of the disclosed brace to resist "riding-up" the spine of the wearer, a trait associated with prior art braces, is believed to be at least partially attributed to primary elastic bands in the core layer strategically skewed relative to the longitudinal or lengthwise direction of the brace. Each band is balanced with an adjacent band having an opposite skew direction. The bands of one skew direction are caused to be further stretched when the brace attempts to ride-up thereby automatically resisting the ride-up movement.

Preferably, the disclosed brace includes an integrated pouch or pocket area for carrying therapeutic aids at the lumbar region such as cold and/or hot packs, reusable or disposable, stimulators and/or magnetic packs.

The invention will now be further described by way of example with reference to the accompanying drawings, in which:

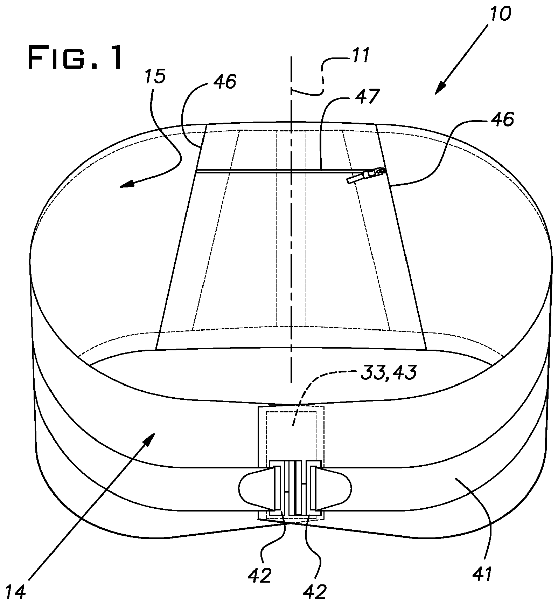

FIG. 1 is a perspective view of the back brace of the invention shown in a worn configuration;
FIG. 2 is a flat view of a rear face of an outer shell of the inventive back brace;
FIG. 3 is a flat view of an inner face of the outer shell of the inventive back brace;
FIG. 3A is an exploded fragmentary cross-sectional view of the brace taken in the plane 3A-3A in FIG. 3;
FIG. 4 is a flat outer view of an inside panel or core of the inventive brace; and
FIG. 4A is a fragmentary cross-sectional view of the core taken in the plane 4A-4A in FIG. 4.

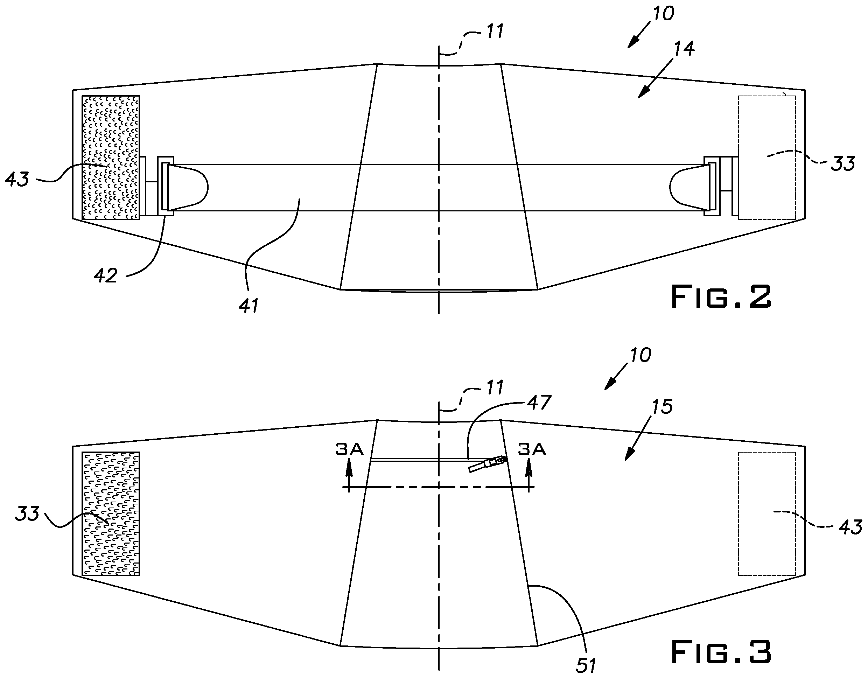

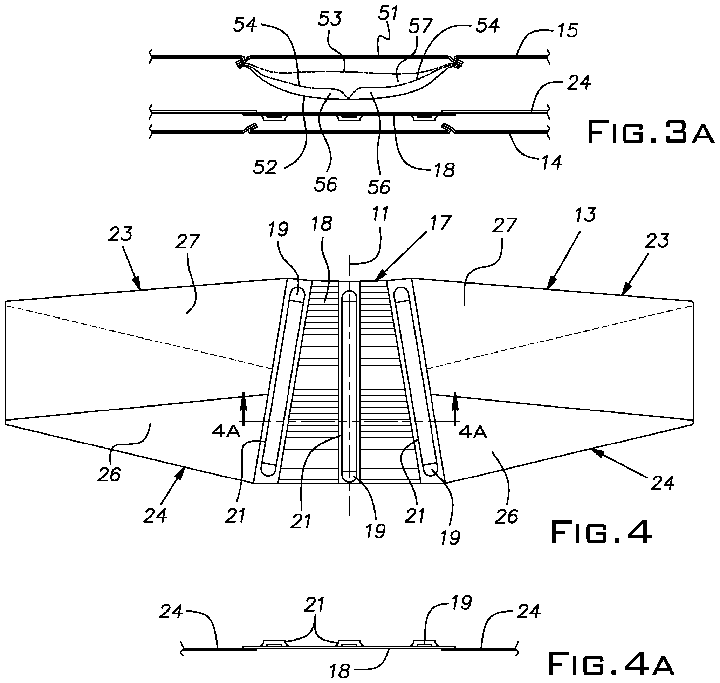

A back brace 10 as depicted when lying flat in a free state has an elongated shape symmetrical about a transverse center line 11. The brace 10 is a composite of three layers, each layer comprising one or more generally flat fabric part(s). The layers include an inner panel or core 13 shown in FIG. 4 and two layers 14, 15, forming an outer shell 16 seen in FIGS. 2 and 3, respectively, each disposed on an opposite side of the core 13.

When the brace 10 is worn by a person, the shell layer 15 is at the inside of the brace 10, normally against the clothing of the person or, less frequently, the person directly. FIGS. 2 - 4 illustrate the respective layers 13-15 of the back brace 10 with an orientation corresponding to that when the brace is worn. That is, the illustrated upper edges of these layers will remain the upper edges during use when worn by a person standing or sitting upright.

The outer shell layer 14 (FIG. 2) is outside, away from the body of the wearer.

The inside core layer or panel 13 (FIG. 4) is symmetrical about the center line 11. A mid or central portion 17 of the core 13 is preferably in the form of an isosceles trapezoid and is constructed of fish line cloth or fabric which is very elastic in the lengthwise direction of the brace 10 but, by virtue of the fish line portion of the fabric running vertically has little or no elasticity in the vertical or transverse direction.

Superposed on the fish line fabric or cloth 18 are a plurality of metal boning pieces or stays 19 each encased in a fabric pouch sewn onto the fish line fabric 18. In the illustrated example, the stays 19 are three in number with a central one in alignment with the vertical center line 11 and outer ones aligned with the side margins of the fish line fabric 18 and diverging from one another in a downward direction. By way of example, the stays or boning members 19 may be flat stainless steel strips of, for example, a thickness of .036 inch (.91 mm), a width of .71 inch (18 mm) and a length of 11 inch (279 mm). The length, in particular, of the stays 19 can be modified to suit the size of the person for which a brace is designed to fit.

Portions 23 of the center panel or core 13 outlying the central zone 17 are constructed of elastic strip material of, for example, 6 inch width. The outlying portions or zones 23 on each side of the central portion 17 can comprise folded elements of a single elastic strip. Each strip, designated 24, is folded on itself at a line distal from the mid or central portion 17 so as to form two strip portions 26, 27 that diverge from one another in a vertical direction. With reference to FIG. 4, portions 26 of the strips 24 have an upward or vertical inclination so as to be skewed relative to a horizontal longitudinal direction while portions 27 of the elastic strips 24 have a downward vertical component so as to be skewed relative to a horizontal direction. Ends of the elastic strips 24 are fixed by sewing to the central region 17 while their folds are fixed by sewing to distal ends of the shell layers 14, 15.

The inner core 13 is encased in the shell 16 formed by the outer and inner layers 14, 15 each having the same peripheral shape as the core. The inner shell layer 15, shown from the inside in FIG. 3 can be constructed of a knit neoprene fabric (i.e. a knit fabric/neoprene/knit fabric laminate) that is very elastic along the longitudinal direction of the brace 10, the stitch courses of the knit fabric being oriented in this direction. A rectangular strip 33 of loop-type fabric fastener such as Velcro® is sewn along one distal end of the exposed face of the layer 15 and to the underlying end of the elastic strip 24 of the core 13.

The outer shell layer 14, shown from the outside in FIG. 2, can also be constructed of a knit neoprene fabric (knit fabric/neoprene/knit fabric laminate) having extensive elasticity in the longitudinal direction of the brace 10 by virtue of the stitch course direction of this knit fabric being oriented in the longitudinal direction of the brace. The layers 14, 15 can be of the same construction if desired, and should exhibit an elasticity in the lengthwise direction of at least 150%.

An elastic belt or cinch 41 across a major part of the length of the brace 10 slightly below a mid-height of the brace is provided on the outer shell layer 14. Opposite ends of the belt 41 are adjustably retained in buckles 42 fixed to the brace 10 adjacent its ends. A strip of hook fabric fastening material 43 such as Velcro® is sewn on the outer shell layer 14 and to the underlying end of the elastic strip 24 of the core 13.

The inside face 15 of the outer shell 16 includes a closure in the form of a zipper 47 that horizontally spans the center or trapezoidal area 51. Referring to FIG. 3A, several internal layers of fabric material are superposed and sewn together along the sides and bottom of the trapezoidal center 51 of the inner shell layer 15. One layer 52 distal from the inner shell layer or face 15 can be of the same material as any of the outer shell proper 16. Two layers 53, 54 can be of elastic net fabric. The net fabric layer 54 is sewn to the distal fabric layer 52 along a vertical line to form adjacent smaller pockets 56. The net fabric layer 53 forms a larger pocket 57 with the net fabric layer 54. Upper edges of the net fabric layers lie vertically below the zipper 47, with the upper edge of the layer 53 being slightly higher than the upper edge of the layer 54. This enables the pockets 56, 57 to be readily accessed through the zipper 47.

The flat zippered pockets 56, 57 can be used for receiving appropriately sized therapeutic articles such as stimulators, reusable ice/cold packs, disposable ice/warm packs, magnets, etc.

The brace 10 is worn by wrapping it about the abdomen so that the central zone 17 overlies the lumbar region of the spine. The ends of the brace 10 are overlapped and with a suitable level of tension obtained, the hook and loop fasteners or mutual coupling elements 43, 36 are pressed and locked together. The level of tension in the brace 10 can be more finely adjusted by tensioning the auxiliary belt 41 in one of the buckles 42.

The brace 10 has a slim or low profile, having a total maximum thickness of 1/4 inch or less even when measured in the multi-ply area of the stays 19. When worn, the brace has a simple, unadorned and uncluttered appearance. All of the seams of the shell are inturned for a smooth, finished appearance.

The neoprene knit fabric of the shell 16 is highly elastic and allows the brace to be capable of conforming to a variety of body contours and positions. By way of example, a typical brace shell having a free girth or length of 35 inches can stretch 2-1/2 inches with the application of 2 pound force. The core 13 having the same dimensions can typically exhibit an extension of 2-1/2 inches with the application of 7 pounds force. The belt 41 is substantially more elastic than the core 13.

The disclosed brace 10 by virtue of its thin, flexible and highly elastic construction, is comfortable and relatively inconspicuous when worn. Consequently, it is more likely to be used and used for longer periods than that of prior known braces.

The knit neoprene fabric forming both the outer and inner layers 14, 15 of the outer shell 16 can, as in conventional contemporary practice, be a composite or laminate of a foam sheet of neoprene sandwiched and bonded between knit fabrics, typically of polyester, nylon, or other suitable synthetic filament. The knit synthetic filament of at least one of the knit fabrics, preferably at least at the inside face of the inner layer 15, i.e. the face towards the wearer, contains pure silver introduced into a filament sheath as it is being co-extruded in the original filament manufacturing process. The antimicrobial silver content is known to destroy odor causing bacteria associated with perspiration. A current source of this silver containing co-extruded filament or thread is Noble Biomaterials Inc. of Scranton, Pennsylvania USA marketed under the Registered Trademark XT2®. Silver bearing fabric technology has been used for years by NASA astronauts, Olympic athletes and the U.S. Special Forces to ensure lasting freshness in the harshest environments. Here, the silver additive can reduce a need to frequently clean the brace, thereby extending its service life and reducing the care needed for its use. Being embedded in the body of the fabric filament, the silver maintains its effectiveness for the life of the brace.

An alternative to the above disclosed neoprene fabric for at least the inner layer 15, and optionally the outer layer 14, is a knit spacer fabric supplied by Noble Biomaterials under the Product Code PET-FT070-0360TFSV Alt Name/Number XT2 290 gsm Spacer Knit with a Fabric Description: Polyester Blend, Knit Circular, Spacer; Content 82% Polyester, 12% Spandex, 6% XT2-Polyester. This knit spacer fabric, as the 6% XT2-Polyester denotes, includes antimicrobial silver carrying filament.

The brace 10 is unusual in its ability to resist a tendency to ride-up a person's torso from the lumbar region when the person sits or otherwise moves about. This ability to maintain its position is believed to be related to the skewed direction of the elastic strip portions 26 relative to the longitudinal direction of the brace 10. A tendency of the central region to ride-up the spine of the wearer is resisted by an increase in vertical downward force in the upwardly inclined elastic strip or band portions 26. Under ordinary conditions, the downwardly inclined elastic strip portions 27 provide a force component that counterbalances the upward inclined strip's downward forces.

The inner layer 15, at least, of the outer shell 16 is soft enough to be worn against or in contact with the skin of the wearer. The antimicrobial function of this layer 15 is most effective when worn against the skin. The brace 10, however, if desired can be worn over clothing.

It should be evident that this disclosure is by way of example and that various changes may be made by adding, modifying or eliminating details.

Embodiments of the invention can be described with reference to the following numbered clauses, with preferred features laid out in the dependent clauses:
1. A back brace comprising a core of elastic sheet material extending in a longitudinal direction and a plurality of relatively rigid narrow, spaced stays transversely attached to a mid-length of the elastic sheet material, the core being substantially completely sheathed by a knit neoprene or spacer fabric shell.
2. A back brace as set forth in clause 1, wherein the neoprene or spacer fabric shell includes a fiber containing antimicrobial silver.
3. A back brace as set forth in either clause 1 or clause 2, wherein the core comprises convergent strips of elastic sheet material spanning a first distance adjacent a longitudinal center of the core and a second distance, less than the first distance, remote from the center.
4. A back brace as set forth in clause 3, wherein the center of the core comprises fish line elastic material.
5. A back brace as set forth in clause 4, wherein the stays are attached on the fish line elastic material.
6. A back brace as set forth in any preceding clause wherein the thickness of major portions of the brace does not substantially exceed 1/4 inch.
7. A back brace comprising a flexible, elastic, elongated panel extending in a longitudinal direction and adapted to be wrapped about the lumbar region and abdomen comprising a central zone and outlying zones on opposite edges of the central zone, the outlying zones each having elastic strip portions lying along directions that are skewed relative to the longitudinal direction such that when the brace is worn the strips are oriented upwardly with increasing proximity to the central zone such that they resist ride-up of the central zone by developing an increase in tension and a downward force component on the central zone.
8. A back brace as set forth in clause 7, wherein the central zone has a plurality of semi-rigid stays attached thereto and oriented generally transverse to the longitudinal direction.
9. A back brace as set forth in either clause 7 or clause 8, wherein the central zone and outlying zones are covered by a knitted fabric layer.
10. A back brace as set forth in clause 9, wherein the knitted fabric layer includes filaments containing antimicrobial silver.
11. A back brace as set forth in either clause 9 or clause 10, wherein the central zone and outlying zones are covered on opposite sides by knitted fabric layers.
12. A back brace as set forth in clause 11, wherein one of the knitted fabric layers serves as an outside layer when the brace is worn, the outside layer forming a pocket area overlying the central zone for carrying therapeutic materials including hot and/or cold packs, magnetic pack, or a stimulator.
13. A back brace as set forth in any one of clauses 6 to 12, wherein the distal ends of the outlying zones have mutually engageable coupling elements.
14. A back brace as set forth in any one of clauses 6 to 13, having a flat configuration in a free state.
15. A back brace as set forth in any one of clauses 6 to 14, including an auxiliary elastic belt disposed on an outside of the brace when the brace is configured to be worn, the ends of the auxiliary belt being attached to the brace adjacent distal ends of the brace.

## Claims

1. A back brace comprising a flexible and elastic elongated panel extending in a longitudinal direction and adapted to be wrapped about the lumbar region and abdomen comprising a core with a central zone and outlying zones on opposite edges of the central zone, the central zone having a plurality of semi-rigid stays attached thereto and oriented generally transverse to the longitudinal direction, the outlying zones each having elastic strip portions lying along directions that are skewed relative to the longitudinal direction such that when the brace is in a worn state, the strips on each side of the central zone are oriented upwardly and downwardly with increasing proximity to the central zone, the core having an inner face and an outer face with reference to the worn state, said core faces each being covered by a knit fabric.

2. A back brace as set forth in claim 1, wherein the knit fabric is a composite including neoprene.

3. A back brace as set forth in either claim 1 or claim 2, wherein the fabric covering the inner core face provides a pocket area for therapeutic packs.

4. A back brace as set forth in claim 3, wherein the pocket area is accessible through a zipper sewn in the fabric covering the inner core face.

5. A back brace as set forth in any preceding claim, wherein said skewed elastic strip portions include portions that decline with increasing proximity to the central zone and tend to counterbalance the downward forces developed by said upwardly inclined strip portions.

6. A back brace as set forth in any preceding claim, wherein said central zone is a fish line elastic fabric.

7. A back brace as set forth in any preceding claim, wherein said central zone includes resilient stays oriented generally perpendicularly to said longitudinal direction.

8. A back brace as set forth in claim 7, wherein said resilient stays are attached to said central zone.

9. A back brace as set forth in any preceding claim, including an adjustable elastic belt having end portions attached to the brace adjacent longitudinal ends of the brace.

10. A back brace as set forth in any preceding claim, wherein the knit fabric includes a fiber carrying antimicrobial silver.
